# EUROPÄISCHE PATENTSCHRIFT

(11) **EP 1 542 605 B1**
(45) Veröffentlichungstag und Bekanntmachung des Hinweises auf die Patenterteilung: **18.06.2008**
(21) Anmeldenummer: 03797968.9
(22) Anmeldetag: 11.09.2003
(51) Int. Cl.: A61B 18/16

(54) **VORRICHTUNG ZUR PRÜFUNG EINER NEUTRALELEKTRODE**
DEVICE FOR EXAMINING A NEUTRAL ELECTRODE
DISPOSITIF DE TEST D'UNE ELECTRODE NEUTRE

(30) Priorität: 24.09.2002 AT 14282002
(43) Veröffentlichungstag der Anmeldung: 22.06.2005
(73) Patentinhaber: NESSLER, Norbert, A-6020 Innsbruck (AT)
(72) Erfinder: NESSLER, Norbert, A-6020 Innsbruck (AT)
(74) Vertreter: Ellmeyer, Wolfgang
(86) Internationale Anmeldenummer: PCT/AT2003/000270
(87) Internationale Veröffentlichungsnummer: WO 2004/028387

(56) Entgegenhaltungen:
- NESSLER N ET AL: "THE NEUTRAL ELECTRODE IN ELECTROSURGERY, A RISK FOR THE PATIENT" BIOMEDICAL INSTRUMENTATION & MEASUREMENT, 12. Juni 2001 (2001-06-12), Seiten 269-272, XP008024524
- NESSLER N ET AL: "Testing Device For Surgical Grounding Plates " ENGINEERING IN MEDICINE AND BIOLOGY SOCIETY, Bd. 6, Seiten 2388-2389, XP002261159 in der Anmeldung erwähnt
- NESSLER N ET AL: "SICHERHEITSTESTER FUER HF-CHIRURGIE-NEUTRALELEKTRODEN (MESSGERAET FUER IMPEDANZ UND TEMPERATURERHOEHUNG UNTER AAMI-HF18 STANDARDLAST) SAFETY TESTING OF HIGH FREQUENCY SURGERY NEUTRAL ELECTRODES (MEASURING INSTRUMENT FOR IMPEDANCE AND TEMPERATURE INCREAS" BIOMEDIZINISCHE TECHNIK, FACHVERLAG SCHIELE UND SCHOEN GMBH. BERLIN, DE, Bd. 38, Nr. 1/2, Januar 1993 (1993-01), Seiten 5-9, XP001119230 ISSN: 0013-5585 in der Anmeldung erwähnt
- GABRIEL S ET AL: "THE DIELECTRIC PROPERTIES OF BIOLOGICAL TISSUES: II. MEASUREMENTS IN THE FREQUENCY RANGE 10 HZ TO 20 GHZ" PHYSICS IN MEDICINE AND BIOLOGY, TAYLOR AND FRANCIS LTD. LONDON, GB, Bd. 41, Nr. 11, 1. November 1996 (1996-11-01), Seiten 2251-2269, XP000635700 ISSN: 0031-9155 in der Anmeldung erwähnt

## Beschreibung

Die Erfindung betrifft eine Vorrichtung zur Prüfung einer Neutralelektrode für elektrochirurgische Anwendungen mit einer aus einer Vielzahl von Meßelektroden gebildeten Meßfläche, auf welche die Neutralelektrode aufbringbar ist, wobei jede Meßelektrode der Meßfläche mit einer zumindest eine Schicht der menschlichen Haut, z.B. die Oberhaut, die Lederhaut und die Unterhaut mit Fettgewebe, repräsentierenden Ersatz-Widerstandschaltung aus zumindest zwei Widerständen verbunden ist, welche Ersatz-Widerstandschaltung mit zumindest jeweils einem Temperaturfühler in thermischem Kontakt steht.

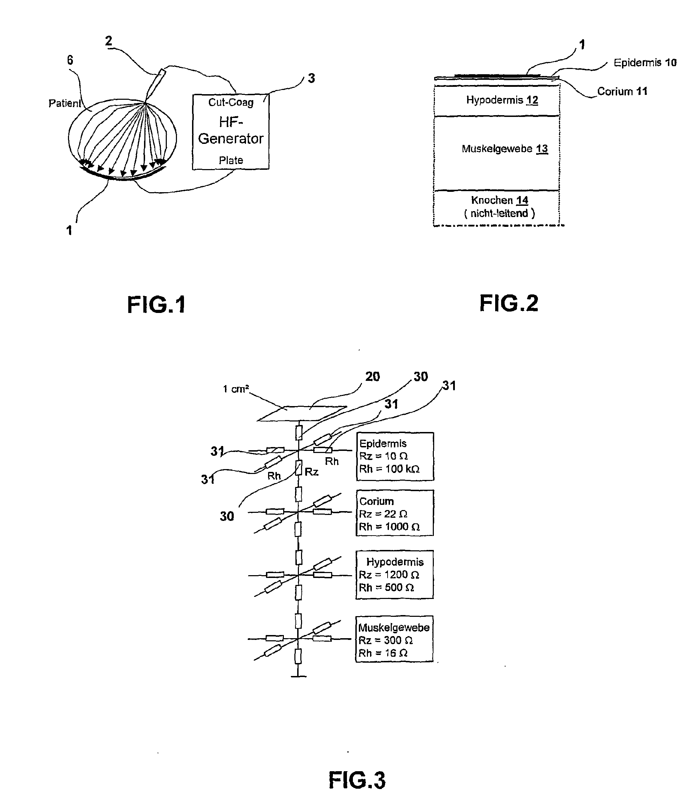

Das Schneiden und Koagulieren von Gewebe durch Anwendung eines hochfrequenten Stromes wird in der Elektrochirurgie bereits seit langem praktiziert. In der sogenannten monopolaren Technik wird, wie in Fig.1 in einer Querschnittdarstellung gezeigt, der HF-Strom zur Erzielung des gewünschten chirurgischen Effekts über eine aktive Elektrode 2 relativ kleiner Fläche, welche über ein Handstück geführt werden kann, in den menschlichen oder tierischen Körper 6 eingeleitet und über eine relativ großflächige Neutralelektrode 1, die zumeist am Oberschenkel, fallweise aber auch am Oberarm oder am Rücken appliziert wird, wieder zu einem HF-Generator 3 zurückgeführt. Zugleich dient die Neutralelektrode 1 auch der Erdung des Patienten. Moderne Neutralelektroden sind selbstklebende, einmal zu verwendende Kontaktflächen unterschiedlicher Größe und Form.

Im Gegensatz zur aktiven Elektrode, bei der die rasche Erwärmung den gewünschten chirurgischen Effekt bewirkt, darf die Neutralelektrode die Haut des Patienten nicht mehr als 6°C erwärmen. Entsprechende Tests sind in der Norm ANSI-AAMI HF 18-93 (American National Standard Institute, Association for the Advancement of Medical Instrumentation, Electrosurgical Devices, Arlington, USA, 1993) festgelegt. Eine Elektrodentype wird dabei an mindestens fünf weiblichen und fünf männlichen Versuchspersonen unter festgelegter Strom-Belastung getestet, die Temperaturmessung erfolgt üblicherweise mit einer Thermo-Kamera. Alternativ dazu ist ein nicht näher beschriebenes, geeignetes Ersatzmedium anstelle der Versuchspersonen zulässig, das nachweislich dazu geeignet ist, die elektrischen und thermischen Eigenschaften der thermisch empfindlichsten Versuchsperson nachzubilden.

Die diesbezüglich an der Universität Innsbruck durchgeführten Untersuchungen (Nessler N., Huter H., Wang L., Testing device for surgical grounding plates, 14th Annual International Conference of the IEEE Engineering in Medicine and Biology Society, Paris, 1992; Huter H., Entwicklung eines Meßgerätes für thermische und elektrische Gleichmäßigkeit von Neutralelektroden der HF-Chirurgie, Diplomarbeit, Universität Innsbruck 1992; Nessler N., Huter H., Wang L., Sicherheitstester für HF-Chirurgie-Neutralelektroden, Biomedizinische Technik Vol.38, p. 5-9, 1993) führten zu einer elektronischen Vorrichtung (GPTest II), welche die Eigenschaften der Haut eines Patienten in erster Näherung soweit nachbildet, daß eine Neutralelektrode unter der in der AAMI HF-18 Norm festgelegten Strombelastung von 700mA während einer Meßzeit von 60 s getestet werden kann. Die dabei entstehende Erwärmung wird gemessen und ergibt mit der Messung an einer Versuchsperson vergleichbare Meßwerte (Nessler N., Skin Temperature Scan with Flexible Sensor Array, 16th BEMS Meeting 1994, Kopenhagen; Nessler N., "Current density distribution in Human skin under the Grounding electrode of Electrosurgery", BEMS 17th Annual Meeting, Boston, MA., 1995). Der durch Volumsleitung in den Hautschichten und Muskelfasern des von der Neutralelektrode überdeckten Gewebes verursachte Randeffekt, der eine Stromkonzentration und damit Erwärmungsmaxima entlang des Randes der Neutralelektrode bewirkt, wird mit der erwähnten Vorrichtung allerdings nicht nachgebildet.

Für die elektronische Nachbildung der Haut in der Vorrichtung GPTest II wurden die Testbedingungen der AAMI HF-18 Norm herangezogen, die eine Bestimmung der Temperaturverteilung über die gesamte Fläche der Neutralelektrode vorsieht, wobei für Bereiche von jeweils 1 cm² die einzelnen Temperaturwerte bestimmt werden. Dazu wird eine Meßfläche, üblicherweise eine Meßplatine mit vergoldeter Kontaktfläche entsprechender Größe, z.B. 10 cm x 18 cm, in eine Vielzahl je 1 cm² großer, quadratischer Meßelektroden unterteilt und zugehörige Durchkontaktierungen auf der Rückseite der Meßplatine über jeweils einen ohmschen Ersatzwiderstand von z.B. 1 kOhm als Mittelwert der Hautimpedanz mit Masse verbunden. Als Temperaturfühler ist für jede Meßelektrode ein in thermischem Kontakt mit dem jeweiligen Ersatzwiderstand stehender Transistor vorgesehen, dessen temperaturabhängige BasisEmitterspannung (-2mV/°C) einen elektronisch auswertbaren Temperaturmeßwert ergibt. Wird nun eine Neutralelektrode auf die in elektrisch voneinander isolierte Meßelektroden unterteilte Meßplatine aufgebracht und mit dem im AAMI HF-18 Standard vorgesehenen Strom von 700 mA 60 s lang belastet, so wird die durch die Vielzahl der mit den Meßelektroden verbundenen Ersatzwiderstände hervorgerufene Temperaturverteilung auf der Neutralelektrode mit Hilfe der Temperaturfühler-Transistoren gemessen und die entsprechenden Basis-Emitterspannungen vor und nach der Heizphase mittels einer elektronischen Schaltung, z.B. in Form eines Matrix-Scans, ausgewertet.

Diese vereinfachte elektronische Darstellung der menschlichen Haut als je ein Widerstandswert pro cm² gegen Masse gibt aber den Randeffekt nicht wieder, der je nach Hautbeschaffenheit und Hautdicke unterschiedliche Stromkonzentrationen am Rand der Neutralelektrode hervorruft, da die in der Haut tatsächlich vorhandenen Querimpedanzen parallel zur Hautoberfläche nicht nachgebildet werden. Die mit dieser Anordnung gemessenen Temperaturen sind daher über die gesamte Fläche der Neutralelektrode im Wesentlichen gleich hoch und ergeben damit eine relativ grobe Abschätzung der für eine Versuchsperson zu erwartenden thermischen Belastung.

Die Stromverteilung innerhalb des Körpers, insbesondere innerhalb des Oberschenkels ist einer direkten Messung nicht zugänglich, zur Ermittlung derselben wurde daher an der Universität Innsbruck eine Modellrechnung durchgeführt (Nessler N., "Die Neutralelektrode bei der Elektrochirurgie, ein Risiko für den Patienten ?", Österr. Krankenhauszeitung ÖKZ, 12/95, p. 29-34,1995; Nessler N., "Current density distribution in Human skin under the Grounding electrode of Electrosurgery", BEMS 17th Annual Meeting, Boston, MA., 1995). Bei dieser als Zylindermodell bezeichneten Berechnung wurde der Oberschenkel mit den verschiedenen Haut- und Muskelschichten nachgebildet und mit der Methode der finiten Elemente der lokale Stromverlauf, der Spannungsverlauf und die lokale Verlustleistung berechnet. Fig. 2 zeigt anhand einer schematischen Schnittdarstellung den angenommenen Schichtaufbau im Körper des Patienten, wobei die Neutralelektrode 1 auf einer Schicht aus Oberhaut (Epidermis) 10 aufliegt, darunter folgt eine Lederhaut-Schicht (Corium) 11, eine Unterhaut-Schicht (Hypodermis) 12 mit Fettgewebe und eine Muskelschicht 13, welche an eine praktisch nicht-leitende Knochenschicht 14 angrenzt. Die berechnete Stromverteilung an der Hautoberfläche entspricht dabei genau dem Erwärmungsbild, das bei Versuchspersonen unter gleichen Bedingungen ermittelt wurde. Diese Modellrechnung zeigt den erwarteten Randeffekt, also eine Stromkonzentration am Rand der Neutralelektrode und weiters eine stärkere Belastung des zur Operationsstelle näheren Teils des Randes der Neutralelektrode, welches Phänomen sich in einer Richtungsabhängigkeit der im Randbereich der Neutralelektrode auftretenden stärkeren Erwärmung bemerkbar macht. Aufgrund dieser Richtungsabhängigkeit erwärmt sich das näher zur Operationsstelle gelegene Gewebe stärker als das weiter entfernt gelegene.

Zur elektronischen Simulation dieses Modells kann in Übereinstimmung mit der durch die AAMI HF-18 festgelegten Unterteilung für die Vielzahl an 1 cm² großen Meßelektroden eine entsprechende Anzahl an Meß-Prismazellen angenommen werden, welche in, die verschiedenen Haut- und Muskelschichten des Patienten nachbildende Teilprismazellen unterteilt sind. Vom Zentrum jeder Teilprismazelle ausgehend ist die jeweilige Haut- oder Muskelschicht durch in drei Raumrichtungen, horizontal in x- und in y-Richtung sowie vertikal in z-Richtung angeordnete Widerstände entsprechend einem Ersatzschaltbild der Impedanzen der Hautschicht dargestellt (Gabriel S., Lau R.W., Gabriel C., "The dielectric properties of biological tissue"; "II Measurements in the frequency range 10 Hz to 20 GHz", Phys. Med. Biol. 41, 2251-2293, 1996). Dieses Modell erfordert pro Schicht sechs Widerstände, woraus sich für Oberhaut 10, Lederhaut 11, Unterhaut 12 mit Fettgewebe und das Muskelvolumen 13 (Fig.2) insgesamt 24 Widerstände pro 1 cm²-Meßelektrode ergeben. Mit den in Fig.2 gezeigten Hautschichten wurde z.B. ein Oberschenkel modelliert und Ströme und damit verbundene Leistungen berechnet. Das daraus resultierende dreidimensionale Ersatzwiderstands-Netzwerk bildet die Eigenschaften der Haut für HF-Strom samt Rand- und Richtungseffekt nach. Ein Prinzipschaltbild des Ersatzwiderstandsnetzwerks für eine Meßelektrode 20 der Meßfläche ist in Fig.3 dargestellt. Jede Hautschicht ist durch jeweils vier Horizontalwiderstände 31 (Rh) und zwei Vertikalwiderstände 30 (Rz) repräsentiert, z.B. Epidermis: Rz= 10 Ohm, Rh = 100 kOhm, Corium: Rz= 22Ohm, Rh =1000 Ohm, Hypodermis: Rz=1200 Ohm, Rh = 500 Ohm, Muskelgewebe: Rz= 300 Ohm, Rh = 16 Ohm. Jeder Horizontalwiderstand 31 ist an einem Anschluß jeweils mit dem Knotenpunkt der Teilprismazelle einer Hautschicht und am anderen Anschluß mit dem Horizontalwiderstand 31 der jeweils benachbarten Teilprismazelle derselben Hautschicht verbunden. Der obere Vertikal-Ersatzwiderstand 30 der Oberhautschicht jeder Meßprismazelle ist an einem Ende direkt mit der Meßelektrode 20 verbunden, während der untere Vertikal-Ersatzwiderstand 30 der Muskelgewebeschicht an einem Ende mit Masse verbunden ist. Die dazwischenliegenden Vertikal-Ersatzwiderstände 30 sind jeweils mit dem Knotenpunkt der zugehörigen Teilprismazelle und dem Vertikal-Ersatzwiderstand der darüberliegenden oder der darunterliegenden Teilprismazelle verbunden.

Die Ersatzwiderstände 30, 31 können selbst bei der Verwendung von sehr kleinen Bauteilen, z.B. SMD, nicht ausreichend dicht gepackt werden, damit 24 Widerstände auf 1 cm² einer Schaltplatine Platz finden. Zur Vereinfachung dieses Modells wurde ein ebenes Modell der Haut mit der Neutralelektrode berechnet, wobei die Bedingung einzuhalten war, daß die Ergebnisse im Wesentlichen die gleichen wie beim Zylindermodell bzw. bei den Messungen an den Versuchspersonen sein mußten. Das gut leitende Muskelvolumen mit seinem großen Querschnitt konnte aufgrund seines geringen ohmschen Widerstandes im Modell durch eine Äquipotentialfläche, also eine Fläche mit unendlich hoher Leitfähigkeit ersetzt werden. Als Nachteil dieser Vereinfachung geht die Richtungsabhängigkeit der Erwärmung der Neutralelektrode zwar verloren, die übrigen Eigenschaften der Haut bleiben dabei aber im Modell erhalten. Durch Variation der Dicke der Unterhaut (schlecht leitendes Fettgewebe) konnte ferner nachgewiesen werden, daß diese Hautschicht sowohl für den Randeffekt selbst als auch für dessen Ausprägung verantwortlich ist.

Einen weiteren bedeutenden Einfluß auf die Stromverteilung im Zusammenhang mit dem Randeffekt hat die Impedanz der Neutralelektrode. Je höher der ohmsche Widerstand der Neutralelektrode ist, desto geringer ausgeprägt ist der Randeffekt, aber auch desto höher die Eigenerwärmung durch den Leistungsabfall an der Neutralelektrode selbst. Moderne, einmal verwendbare, selbstklebende Neutralelektroden haben eine Impedanz von unter 200 Ohm/cm², eine merkliche Eigenerwärmung tritt jedoch erst ab 2000 Ohm/cm² auf.

Die elektronische Simulation des vereinfachten ebenen Modells ermöglicht weitere Vereinfachungen des Widerstandsnetzwerks. Dies wird dadurch erreicht, daß Widerstände in Serie kombiniert und vernachlässigbare Widerstände eliminiert werden. Z.B. kann der Horizontal-Ersatzwiderstand 31 (Rh) der Epidermis (100 kOhm) gegen den Horizontal-Ersatzwiderstand 31 (Rh) der an diese angrenzenden Lederhaut (1 kOhm) vernachlässigt und somit eliminiert werden. Jeder Schritt einer weiteren Vereinfachung muß daraufhin geprüft werden, ob auch nach diesem Schritt die geforderten Eigenschaften in Übereinstimmung mit dem vollständigen Zylindermodell und mit den Meßergebnissen an Versuchspersonen stehen.

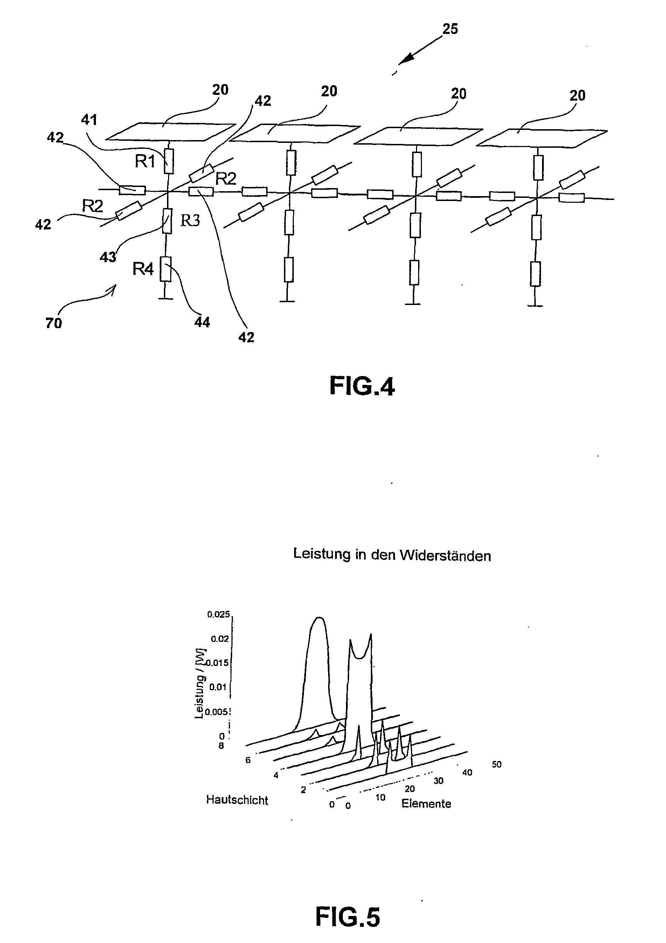

Um nicht jeden Zwischenschritt experimentell aufbauen zu müssen, wurde die Widerstandskombination mittels des Programmes Matlab simuliert und die Ströme, Spannungen und Verlustleistungen in jedem Zweig berechnet. Das Ergebnis der Vereinfachung ist eine Meßfläche 25, bei der jede Meß-Prismazelle durch eine Kombination von sieben Widerständen gebildet ist, wie sie in Fig.4 gezeigt sind.

Dabei wurde der Horizontal-Ersatzwiderstand 31 (Rh) der Oberhaut (Epidermis) des in Fig.3 gezeigten Modells eliminiert, Ersatzwiderstand 41 (R1) ist die Kombination des Vertikal-Ersatzwiderstandes 2 x Rz der Oberhaut mit dem Vertikal-Ersatzwiderstand Rz der Lederhaut, die Ersatz-Widerstände 42 (R2) entsprechen den Horizontal-Ersatzwiderständen 31 (Rh) der Lederhaut. Ersatzwiderstand 43 (R3) ist der verbleibende Vertikal-Ersatzwiderstand Rz der Lederhaut und Ersatzwiderstand 44 (R4) stellt den Vertikal-Ersatzwiderstand der Unterhaut dar. Der Horizontal-Ersatzwiderstand Rh der Unterhaut wurde vernachlässigt. Die an den Ersatzwiderstand 44 (R4) anschließende gemeinsame Masse entspricht dem gut leitenden Muskelvolumen. Die daraus sich ergebende, durch Matlab berechnete Verteilung der Verlustleistung in den simulierten Hautschichten des vereinfachten Ersatzwiderstandsmodells ist in Fig.5 gezeigt. Aus dieser sowie aus der Modellrechnung ergibt sich, daß die Lederhaut die wesentliche Wärmequelle darstellt. Die in Fig.5 als "Elemente" bezeichnete Größe entspricht dabei den durch Ersatzwiderstände gebildeten Meßprismazellen. Die Verlustleistung in der Unterhaut verteilt sich auf ein wesentlich größeres Volumen, die resultierende Temperaturerhöhung ist damit deutlich geringer als in der Lederhaut.

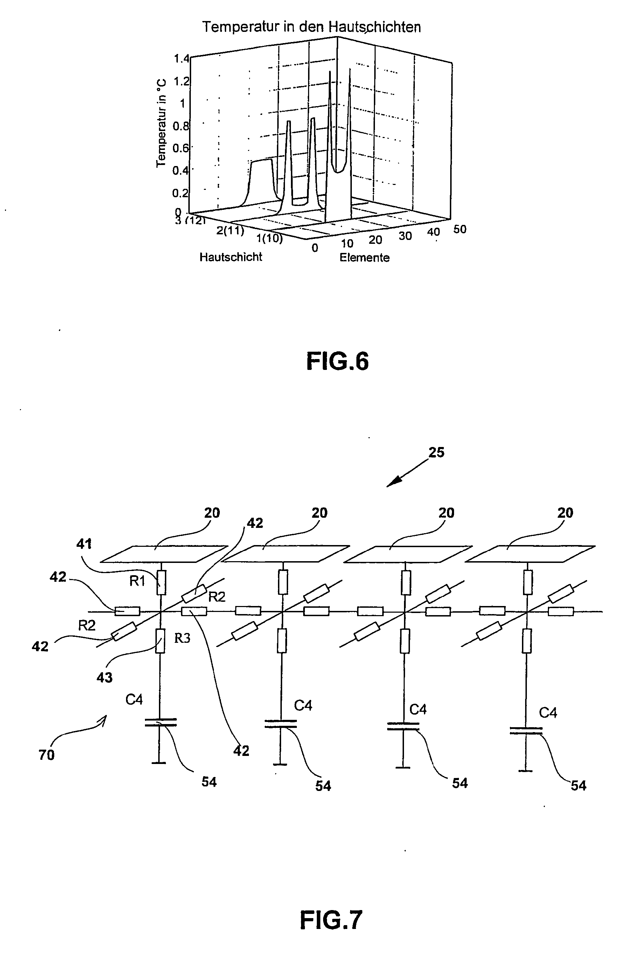

Aus der berechneten Verlustleistungsverteilung ist eine Temperaturabschätzung in den Hautschichten unter der Annahme, daß im wesentlichen Wasser in der Haut aufgeheizt wird, möglich. Der Umrechnungsfaktor zwischen Energieverlust (Produkt aus Leistung und Zeit) und Erwärmung (°C) beträgt 4,193 °C/Ws, ein entsprechendes Diagramm der Temperaturverteilung in Abhängigkeit von der Hautschicht und von den Elementen ist in Fig.6 dargestellt. Hautschicht 1 (10) ist darin die Oberhaut, Hautschicht 2(11) die Lederhaut und Hautschicht 3(12) die Unterhaut.

Die Temperaturerhöhung zeigt den erwünschten Randeffekt, der in der Lederhaut 11 deutlich breiter ist als in der Oberhaut 10 (Fig.2). Die Erwärmung der Unterhaut 12 spielt eine untergeordnete Rolle, da diese sich auf ein wesentlich größeres Volumen verteilt. Zum Vergleich hat die Lederhaut 11 eine durchschnittliche Dicke von 1,5 mm und die Unterhaut eine durchschnittliche Dicke von 10 bis 30 mm.

In der tatsächlich realisierten Ersatz-Widerstandschaltung wird aber nicht ein Wasservolumen aufgeheizt, sondern der jeweils für dieses Volumen repräsentative Ersatz-Widerstand. Für das pro Meßelektrode anzunehmende Volumen der Lederhaut (1 cm² x 1,5mm) muß die am Ersatz-Widerstand gemessene Temperaturerhöhung mit einem Umrechnungsfaktor von 1,73 multipliziert werden, um die Temperaturerhöhung des Wasservolumens bei gleicher Energie zu erhalten.

Im gerechneten Modell (Zylindermodell und ebenes Modell) kann ebenso wie im Experiment mit Versuchspersonen gezeigt werden, daß der Energieverlust in der Unterhaut, der über die Fläche der Neutralelektrode etwa konstant ist (Fig.6), in der resultierenden Erwärmung wegen des großen Volumens dieser Hautschicht keine Rolle spielt. Dieser Effekt ist für die Unterhaut deutlich festzustellen, kann aber auch in anderen Hautschichten zur Wirkung kommen.

Eine Möglichkeit, diesen Effekt zu simulieren, besteht darin, den Ersatzwiderstand insbesondere für die Unterhaut vorzusehen, diesen Ersatzwiderstand aber außerhalb des räumlichen Meßbereiches des der jeweiligen Meßelektrode zugehörigen Temperaturmeßfühlers anzuordnen, sodaß dieser keinen Beitrag zur Erwärmung leisten kann. In der Praxis führt dies aber zu einer Vielzahl von Leitungen im Randbereich der Meßfläche und kann nur mittels herstellungstechnisch aufwendiger und daher kostenintensiver Multilayer-Platinen realisiert werden.

Weiters ist in NESSLER N et al: "The neutral electrode in electrosurgery, a risk for the patient" Biomedical Instrumentation & Measurement, 12. Juni 2001 (2001-06-12), Seiten 269-272, XP008024524 eine Vorrichtung zur Prüfung einer Neutralelektrode mit einer Vielzahl von Meßelektroden beschrieben, bei der jede Meßelektrode der Meßfläche mit die Schichten der menschlichen Haut repräsentierenden Ersatz-Widerstandsschaltungen verbunden ist. In diesem Dokument wird ferner auf den Artikel GABRIEL S et al: "The dielectric properties of biological tissues: II. measurements in the frequency range 10 Hz to 20 GHz" Physics in medicine and biology, Taylor and Francis Ltd. London, GB, Bd. 41, Nr. 11, 1. November 1996 (1996-11-01), Seiten 2251-2269, XP000635700 ISSN: 0031-9155 hingewiesen, worin die dielektrischen Eigenschaften von Gewebe in Form eines Leitwerts und einer Permitivität beschrieben werden.

Aufgabe der Erfindung ist es, eine Vorrichtung der eingangs genannten Art anzugeben, mit der die in einer Hautschicht auftretende Verlustleistung simuliert werden kann, ohne daß diese zur Gesamterwärmung der einzelnen Meßprisma-Zellen beiträgt.

Erfindungsgemäß wird dies dadurch erreicht, daß zumindest eine Hautschicht innerhalb der Ersatz-Widerstandschaltung von einem Blindwiderstand repräsentiert wird.

Durch die Realisierung zumindest eines Widerstandes der Ersatz-Widerstandschaltung als Blindwiderstand entsteht bei Anlegen einer Wechselspannung an die Ersatz-Widerstandschaltung keine Verlustleistung am Blindwiderstand. Folglich kommt es auch nicht zu einer Erwärmung desselben. Der Blindwiderstand kann daher innerhalb des unmittelbaren Bereiches der Meßelektrode und des zugehörigen Temperaturfühlers angeordnet werden. Zusätzlich läßt sich der Wert des Blindwiderstandes durch Wahl der Frequenz der an die Ersatz-Widerstandschaltung angelegten Spannung verändern. Damit kann in einer Simulation die Impedanz der jeweiligen Hautschicht, für die ein Blindwiderstand vorgesehen wurde, und damit deren Dicke auf einfache Weise durch Variation der Frequenz geändert werden.

Als Blindwiderstand kann grundsätzlich eine Induktivität, eine Kapazität oder eine Kombination derselben vorgesehen sein. Ein hoher Miniaturisierungsgrad läßt sich gemäß einer Weiterbildung der Erfindung am besten durch das Vorsehen eines Kondensators als Blindwiderstand herbeiführen.

Da die Unterhaut gegenüber den anderen Hautschichten eine relativ hohe Dicke aufweist und daher insgesamt nur einen unbedeutenden Anteil an der Gesamterwärmung aller Hautschichten unter der Neutralelektrode hat, kann gemäß einer bevorzugten Ausführungsform der Erfindung der die Unterhaut repräsentierende Widerstand der Ersatz-Widerstandschaltung durch den Blindwiderstand gebildet sein.

Eine Simulation unterschiedlicher Hautdicken läßt sich durch Veränderung der Frequenz der bei aufgebrachter Neutralelektrode mit dieser verbundenen Wechselspannungsquelle erreichen, weshalb gemäß einer weiteren Ausbildung die Wechselspannungsquelle variable Frequenz aufweist.

Es hat sich weiters als vorteilhaft erwiesen, die Meßelektroden mit den ihr zugeordneten Ersatz-Widerstandschaltungen in Form einer Matrix mit Spalten und Zeilen anzuordnen. Die Vorteile einer solchen Matrixanordnung liegen in der Vereinfachung der Temperaturmessung durch Matrix-Scan der Temperaturfühler und im relativ einfach herstellbaren Layout der Meßfläche.

Bei Anwendung des erfindungsgemäßen Prinzips auf das Ersatzwiderstand-Modell mit sechs Widerständen weist gemäß einer weiteren Ausführungsform der Erfindung jede Ersatz-Widerstandschaltung einen zentralen Knotenpunkt auf, der jeweils mit dem einen Anschluß von vier Horizontal-Ersatzwiderständen und mit dem einen Anschluß von einem ersten und einem zweiten Vertikal-Ersatzwiderstand verbunden ist, wobei das andere Ende der Horizontal-Ersatzwiderstände jeweils mit dem Horizontal-Ersatzwiderstand der in Spalten- und Zeilenrichtung benachbarten Ersatz-Widerstandschaltungen verbunden ist, und wobei der andere Anschluß des ersten Vertikal-Ersatzwiderstandes mit der Meßelektrode und der andere Anschluß des zweiten Vertikal-Ersatzwiderstandes über den Blind-Widerstand mit Masse verbunden ist. In dieser Ausführungsform der Erfindung ist somit in dem auf sieben Widerstände reduzierten Modell (Fig.4) der Vertikal-Ersatzwiderstand der Unterhaut durch den Blind-Widerstand ersetzt worden, der damit zur Gesamterwärmung jeder Meßprisma-Zelle keinen Beitrag mehr liefert.

Da bei Versuchsmessungen an Patienten eine über die Berandung der Neutralelektrode hinausgehende Erwärmung festgestellt worden ist, wurde diese durch zusätzliche Ersatzwiderstände simuliert, die allerdings nicht unmittelbar mit den Meßelektroden der Meßfläche verbunden sind. Um dies zu erreichen, können gemäß einer weiteren Ausführungsform der Erfindung die den entlang des Randes der Meßfläche angeordneten Meßelektroden zugeordneten Ersatz-Widerstandschaltungen jeweils mit einer Randimpedanzschaltung mit zumindest einem zugeordneten Temperaturfühler verbunden sein, wobei die Randimpedanzschaltung jeweils mit dem Anschluß des randseitig offenen Horizontal-Ersatzwiderstandes der der am Rand der Meßfläche gelegenen Meßelektrode zugeordneten Ersatz-Widerstandschaltung verbunden ist.

Es sind daher außerhalb der eigentlichen Meßfläche noch weitere Ersatzwiderstands-Elemente mit Temperaturmeßfühlern anzubringen, um eine unbegrenzte Darstellung des Volumsleiters zu ermöglichen. Diesbezüglich kann eine weitere Ausführungsform der Erfindung darin bestehen, daß jede Randimpedanzschaltung aus einer oder mehreren, vorzugsweise zwei, nacheinander geschalteten Rand-Zellen gebildet ist, wobei die Randzelle(n) jeweils einen zentralen Knotenpunkt aufweist bzw. aufweisen, der jeweils mit dem einen Anschluß von vier Horizontal-Randwiderständen, die jeweils gleich den Horizontal-Ersatzwiderständen sind, und mit dem einen Anschluß von einem Vertikal-Randwiderstand, der gleich dem zweiten Vertikal-Ersatzwiderstand ist, verbunden ist, wobei das andere Ende der Horizontal-Randwiderstände jeweils mit dem Horizontal-Ersatzwiderstand bzw. dem Horizontal-Randwiderstand der in Spalten- und Zeilenrichtung benachbarten Ersatz-Widerstandschaltungen bzw. Randzellen verbunden ist, und wobei der andere Anschluß des Vertikal-Randwiderstandes über den Blind-Widerstand mit Masse verbunden ist.

Die Haut des Patienten erstreckt sich aber über den unmittelbaren Randbereich der Neutralelektrode, in dem ein Erwärmungseffekt gemessen werden kann, hinaus und dieses außerhalb des unmittelbaren Neutralelektrodenbereiches vorhandene Hautvolumen hat ebenso einen wenn auch geringeren Stromfluß zur Folge,wie jenes unmittelbar unter der Neutralelektrode liegende, der zwar für die Erwärmung der Haut keine Rolle spielt, dennoch aber einen Einfluß auf die Gesamtstromverteilung hat. Dieser zusätzliche Stromanteil könnte durch weitere Aneinanderschaltung von Randzellen simuliert werden. Um aber die Abmessungen der Meßplatine möglichst klein zu halten, kann vorgesehen sein, daß die Randimpedanzschaltung über eine Abschlußimpedanzschaltung mit Masse verbunden ist. In bevorzugter Weise wird die Abschlußimpedanzschaltung so dimensioniert, daß der Strom von 15 bis 20 Randzellen durch diese fließt, wodurch gewährleistet ist, daß in der Simulation eine relativ große Fläche um den Neutralelektrodenbereich herum berücksichtigt wird.

Wie bereits erwähnt, geht durch das Vernachlässigen der Horizontal-Ersatzwiderstände der Muskelgewebeschicht in der Ersatz-Widerstandschaltung die Richtungsabhängigkeit der Temperaturverteilung verloren. Eine elektronische Umsetzung hätte vier weitere Ersatzwiderstände pro Meßprisma-Zelle zur Folge. Allerdings lassen sich diese aufgrund von Symmetrieüberlegungen auf einen einzigen Widerstand für jede Meßprisma-Zelle reduzieren, wobei gemäß weiterer Ausbildung der Erfindung für jede Zeile oder für jede Spalte der Matrix aus Meßelektroden und zugeordneten Ersatz-Widerstandschaltungen jeweils ein gemeinsamer, die Muskelschicht repräsentierender Horizontal-Ersatzwiderstand vorgesehen sein kann, der außerhalb des räumlichen Meßbereiches der Temperaturfühler angeordnet ist.

Ein vorteilhafte schaltungstechnische Variante der erfindungsgemäßen Vorrichtung kann dabei darin bestehen, daß ein Anschluß der gemeinsamen Horizontal-Ersatzwiderstände jeweils mit den den Meßelektroden der Zeile oder der Spalte zugeordneten Ersatz-Widerstandschaltungen verbunden ist. Dadurch ergibt sich eine wesentliche Vereinfachung des Layouts der Verbindungsleitungen.

Moderne Generatoren für die Elektrochirurgie beinhalten eine Meßschaltung, mit der bei Verwendung einer geteilten Neutralelektrode die Kontaktqualität zwischen der Haut des Patienten und der Neutralelektrode während der Operation gemessen werden kann (CQM). Dabei wird beispielsweise eine Spannung an die beiden Hälften der Neutralelektrode angelegt und die daraus resultierende Impedanz bestimmt. Eine Erhöhung dieses Impedanzwertes über einen vorbestimmbaren Schwellenwert hinaus, z.B. wegen Ablösung eines Teils der Neutralelektrode von der Haut des Patienten löst im elektrochirurgischen Gerät einen Alarm aus. Auf diese Weise wird die Verbrennungsgefahr durch teilweises Ablösen der Neutralelektrode von der Haut des Patienten vermieden. Der Funktionstest für diese Überwachungsschaltung mußte bisher ebenfalls an Versuchspersonen durchgeführt werden. Aufgabe der Erfindung ist es daher, einen solchen Funktionstest nicht direkt am Menschen sondern an einer Vorrichtung mit genau vorherbestimmbaren Prüfbedingungen durchführen zu können.

Erfindungsgemäß wird dies durch die Verwendung einer erfindungsgemäßen Vorrichtung zur Überprüfung der Neutralelektroden-Kontaktqualitätsüberwachungsschaltung (QCM) eines elektrochirurgischen Geräts gelöst.

Mit der durch die erfindungsgemäße Vorrichtung verwirklichten Hautsimulation ist die Überprüfung einer QCM-Schaltung unter kontrollierten Bedingungen möglich, wobei die Ersatz-Widerstandschaltungen der Meßelektroden und gegebenenfalls die Abschlußimpedanzschaltungen zusammen die Prüfimpedanz ergeben. Dabei wird die erfindungsgemäße Vorrichtung passiv, also ohne Anlegen einer Spannung zwischen Neutralelektrode und dem Widerstandsnetzwerk der Ersatz-Widerstandschaltungen betrieben.

Weiters betrifft die Erfindung eine Vorrichtung zur Prüfung einer Neutralelektrode für elektrochirurgische Anwendungen mit einer aus einer Vielzahl von Meßelektroden gebildeten Meßfläche, auf welche die Neutralelektrode aufbringbar ist, wobei jede Meßelektrode der Meßfläche mit einer zumindest eine Schicht der menschlichen Haut, z.B. die Oberhaut, die Lederhaut und die Unterhaut mit Fettgewebe, repräsentierenden Ersatz-Widerstandschaltung aus zumindest zwei Widerständen verbunden ist, wobei die Ersatz-Widerstandschaltung mit zumindest jeweils einem Temperaturfühler in thermischem Kontakt steht, und wobei die Meßelektroden auf einer Seite einer die Meßfläche bildenden Meßplatine ausgebildet und die Widerstände der zugeordneten Ersatz-Widerstandschaltungen auf der gegenüberliegenden Seite der Meßplatine angeordnet sind.

Bisher verwendete Vorrichtungen dieser Art weisen den Nachteil auf, daß die auf der Meßplatine vorgesehenen Ersatz-Widerstände zwischen den ebenfalls auf der Meßplatine angeordneten Temperaturfühlern ihre Verlustleistung auch in Richtung der Temperaturfühler der benachbarten Meßprismazelle abstrahlen und es daher zu Fehlmessungen kommt.

Aufgabe der Erfindung ist es, eine vorstehend genannte Vorrichtung anzugeben, mit der die durch die Ersatz-Widerstandschaltungen hervorgerufene Erwärmung für jede Meßzelle ohne Beeinflussung durch die jeweils benachbarten Ersatz-Widerstandschaltungen möglichst präzise gemessen werden kann.

Erfindungsgemäß wird dies dadurch erreicht, daß die Temperaturfühler auf einer weiteren Platine angeordnet sind, welche von der Meßplatine durch eine mit Durchbrechungen versehene Lochrasterplatte beabstandet ist, wobei die den Widerständen der Ersatz-Widerstandschaltungen zugekehrten Temperaturfühler in die Durchbrechungen der Lochrasterplatte ragen.

Auf diese Weise sind die Temperaturfühler jeweils den betreffenden Ersatz-Widerstandschaltungen gegenüberliegend angeordnet und durch die Lochrasterplatte definiert beabstandet. Der Einfluß der Nachbar-Meßprisma-Zellen auf die Bestimmung der Zellentemperatur läßt sich damit stark herabsetzen.

Bei matrixartiger Anordnung der Meßelektroden können die Durchbrechungen der Lochrasterplatte gemäß einer Weiterbildung der Erfindung konzentrisch zu den Meßelektroden-Mittelpunkten angeordnet sein.

Die erwünschte Wärmeleitung zwischen den Temperaturfühlern und den jeweiligen Ersatzwiderstandschaltungen läßt sich gemäß einer weiteren Ausführungsform der Erfindung deutlich steigern, wenn der innerhalb der Durchbrechungen bestehende Raum zwischen den Widerständen der Ersatz-Widerstandschaltungen und den Temperaturfühlern mit einem wärmeleitenden Material gefüllt ist.

Eine ebenso erwünschte Trennung benachbarter Meßprisma-Zellen gegen Wärmetransport läßt sich hingegen erreichen, wenn der Zwischenraum zwischen den Widerständen der Ersatz-Widerstandschaltungen mit einem wärmeisolierenden Material gefüllt ist.

Eine sehr weitgehende Miniaturisierung der für den Aufbau der erfindungsgemäßen Vorrichtung erforderlichen Bauelemente kann weiters erzielt werden, wenn die Widerstände der Ersatz-Widerstandschaltungen und die Temperaturfühler durch SMD-Bauelemente gebildet sind.

Auch eine sehr dichte Packung der Ersatzwiderstände gehört zu jenen Maßnahmen, die eine Verbesserung der temperaturmäßigen Trennung der einzelnen Meßprisma-Zellen bewirkt. Eine Variante der Erfindung kann daher darin bestehen, daß die Widerstände der Ersatz-Widerstandschaltungen im unmittelbaren Bereich der zugehörigen Meßelektrode auf der gegenüberliegenden Seite der Meßplatine angeordnet und über jeweils zumindest eine Durchkontaktierung mit dieser verbunden sind.

Nachfolgend wird die Erfindung anhand der in den Zeichnungen dargestellten Ausführungsbeispiele eingehend erläutert. Es zeigt dabei
Fig.7 einen Teil einer Schaltungsanordnung für eine Ausführungsform der erfindungsgemäßen Vorrichtung;
Fig.8 einen Teil einer Schaltungsanordnung für eine weitere Ausführungsform der erfindungsgemäßen Vorrichtung;
Fig.9 einen Teil einer Schaltungsanordnung einer weiteren Ausführungsform der erfindungsgemäßen Vorrichtung und einen Ausschnitt aus einem zugehörigen Layout für eine Meßplatine;
Fig.10 einen Ausschnitt aus einem Layout für eine Meßplatine einer weiteren Ausführungsform der erfindungsgemäßen Vorrichtung;
Fig.11 einen Ausschnitt aus einem Layout für die Meßplatine gemäß Fig.10 sowie für eine weitere Platine mit Temperaturfühlern;
Fig.12 einen Schnitt durch einen Teil der in Fig.10 und 11 dargestellten Ausführungsform der erfindungsgemäßen Vorrichtung und
Fig.13 einen Teil einer Schaltungsanordnung einer weiteren Ausführungsform der erfindungsgemäßen Vorrichtung;

Fig.7 zeigt eine Vorrichtung zur Prüfung einer nicht dargestellten Neutralelektrode für elektrochirurgische Anwendungen, etwa wie sie in Zusammenhang mit der Anordnung in Fig.1 beschrieben ist, mit einer aus einer Vielzahl von Meßelektroden 20 gebildeten Meßfläche 25, auf welche die Neutralelektrode aufbringbar ist. Die Meßelektroden 20 sind dabei mit den ihr zugeordneten Ersatz-Widerstandschaltungen 70 vorzugsweise in Form einer Matrix mit Spalten und Zeilen angeordnet, wobei in Fig.7 nur ein Teil einer Zeile dargestellt ist.

Erfindungsgemäß ist zumindest ein Widerstand jeder Ersatz-Widerstandschaltung 70 durch einen Blindwiderstand 54 gebildet.

Jede Ersatz-Widerstandschaltung 70 gemäß Fig.7 weist bevorzugt einen zentralen Knotenpunkt auf, der jeweils mit dem einen Anschluß von vier Horizontal-Ersatzwiderständen 42 und mit dem einen Anschluß von einem ersten und einem zweiten Vertikal-Ersatzwiderstand 41, 43 verbunden ist. Das andere Ende der Horizontal-Ersatzwiderstände 42 ist jeweils mit dem Horizontal-Ersatzwiderstand 42 der in Spalten- und Zeilenrichtung benachbarten Ersatz-Widerstandschaltungen 70 verbunden, der andere Anschluß des ersten Vertikal-Ersatzwiderstandes 41 ist hingegen mit der Meßelektrode 20 und der andere Anschluß des zweiten Vertikal-Ersatzwiderstandes 43 über den Blind-Widerstand 54 mit Masse verbunden. Natürlich kann die Zusammenschaltung und Dimensionierung der Widerstände der Ersatz-Widerstandschaltung 70 variieren, die Umsetzung des erfindungsgemäßen Prinzips ist durch den Blind-Widerstand 54 gegeben.

Eine mögliche Variante zur Ausbildung der Meßfläche 25 besteht darin, die Meßelektroden 20 als 1 cm² große, vorzugsweise quadratische, voneinander elektrisch isolierte Bereiche auf einer in Fig.7 nicht dargestellten Meßplatine auszubilden und die Meßelektroden 20 jeweils mit Durchkontaktierungen zu versehen, über die jede Meßelektrode 20 der Meßfläche 25 mit der zumindest eine Schicht der menschlichen Haut, z.B. die Oberhaut, die Lederhaut und die Unterhaut mit Fettgewebe, repräsentierenden Ersatz-Widerstandschaltung 70 verbunden ist, die mit zumindest jeweils einem, in Fig.7 nicht gezeigten Temperaturfühler in thermischem Kontakt steht. Die Ersatz-Widerstände 41, 42, 43, 54 der Ersatz-Widerstandschaltung 70 sind vorzugsweise als SMD-Bauelemente ausgebildet, die auf der den Meßelektroden 20 abgewandten Seite der Meßplatine aufgelötet und über die vorgesehenen Durchkontaktierungen mit den Meßelektroden 20 verbunden sind. Jeder andere, für den Fachmann gleichwertige Aufbau der Meßfläche 25 mit den Ersatz-Widerständen 41, 42, 43, 54 kann ebenso eine Grundlage für weitere Ausführungsformen der Erfindung bieten.

Bevorzugt sind die mit den Ersatz-Widerstandschaltungen in thermischem Kontakt stehenden Temperaturfühler in an sich bekannter Weise durch Bipolartransistoren gebildet.

Beim Ausführungsbeispiel gemäß Fig.7 ist der in Fig.4 vorgesehene Ersatzwiderstand 44 der Ersatz-Widerstandschaltung 70 durch einen Kondensator 54 ersetzt, dessen Blindwiderstandswert von der Frequenz abhängig ist und Rc= 1/(j.w.C) beträgt, also verkehrt proportional zur Kapazität und zur Frequenz eines angelegten Wechselsignals ist (ω = 2π.f). Der Kondensator 54 ersetzt im Ausführungsbeispiel gemäß Fig.7 somit als Blindwiderstand den ohmschen Widerstand 44, der im Ausführungsbeispiel gemäß Fig.4 die Unterhaut-Schicht 12 (Fig.2) jeder Meßprisma-Zelle repräsentiert und erzeugt innerhalb der Ersatz-Widerstandschaltung 70 keine Verlustwärme, weshalb der Kondensator 54 auch im Bereich des nicht dargestellten Temperaturfühlers angeordnet sein kann, ohne daß er zur Erwärmung desselben beiträgt. Der Kondensator 54 kann somit wie die anderen Ersatzwiderstände 41, 42, 43 jeder Meßprisma-Zelle in Form eines SMD-Bausteins auf der Meßplatine angeordnet sein.

Durch Veränderung der Frequenz des eingespeisten Stromes kann der Blindwiderstandswert des Kondensators 54 geändert und so verschiedene Dicken der Unterhautschicht 12 (Fig.2) simuliert werden. Es ist daher vorteilhaft, wenn über eine Wechselspannungsquelle variabler Frequenz eine Wechselspannung zwischen Neutralelektrode und den Ersatz-Widerstandschaltungen 70 angelegt wird, um so durch Variation der angelegten Wechselspannung eine unterschiedliche Dicke der Unterhautschicht 12 simulieren zu können.

Die Kapazität des Kondensators 54 ist mit Rücksichtnahme auf die Frequenz der Wechselspannungsquelle, mit der die erfindungsgemäße Vorrichtung betrieben wird, zu bestimmen. Anstelle des Kondensators 54 kann im Rahmen der Erfindung auch eine Induktivität als Blindwiderstand in der Ersatz-Widerstandschaltung 70 vorgesehen sein.

Es können weiters auch andere Schichten als die Unterhautschicht 12 (Fig.2) durch das Vorsehen eines Blindwiderstandes in der Ersatzwiderstandschaltung simuliert werden. Auch ist eine Anwendung der Erfindung, insbesondere das Ersetzen eines ohmschen Widerstandes durch einen Blindwiderstand genauso bei einer anderen schaltungsmäßigen Anordnung von Ersatzwiderständen als der in Fig.4 und Fig.7 gezeigten möglich.

Die in den Fig.7 erkennbare Quervernetzung der einzelnen Teilprismazellen über die Horizontal-Ersatzwiderstände 42 als Simulation der Lederhautschicht 11 (Fig.2) bewirkt entsprechende Querströme und in Übereinstimmung mit der zugrunde liegenden Modellrechnung einen ausgeprägten Randeffekt, der durch die Beeinflussung der Frequenz der an die Ersatz-Widerstandschaltung 70 über die Neutralelektrode und die Meßelektroden 20 angelegten Spannung veränderbar ist, da sich die Impedanz des die Unterhaut 12 repräsentierenden Blindwiderstandes 54 in Abhängigkeit von der Frequenz ändert, sodaß sich für unterschiedliche Unterhautdicken ein anderer Randeffekt ergibt.

Die durch den Randeffekt bewirkte Erwärmung im Bereich der Randzone der Neutralelektrode reicht beim Patienten über die Fläche der Neutralelektrode 1 hinaus, weshalb in der AAMI HF-18 Norm eine Temperaturmessung auf einer über die Neutralelektrodenfläche hinausgehenden Fläche vorgeschrieben ist. Für die elektronische Simulation bedeutet dies, daß im Randbereich der Neutralelektrode 1, außerhalb der eigentlichen Meßfläche zusätzliche Ersatzwiderstände anzubringen sind, um den Volumsleiter quasi unbegrenzt darzustellen.

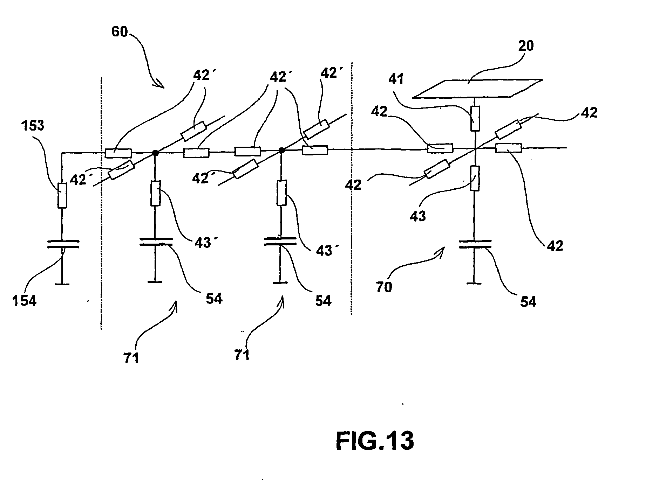

Zu diesem Zweck sind die den entlang des Randes der Meßfläche 25 angeordneten Meßelektroden 20 zugeordneten Ersatz-Widerstandschaltungen 70 jeweils mit einer Randimpedanzschaltung 60 verbunden, wie dies in Fig.13 dargestellt ist. Zur Messung der durch die Randimpedanzschaltung 60 hervorgerufenen Erwärmung ist weiters zumindest ein dieser zugeordneter Temperaturfühler vorgesehen.

Die Abschlußimpedanzschaltung 60 ist dabei jeweils mit dem Anschluß des randseitig offenen Horizontal-Ersatzwiderstandes 42 der der am Rand der Meßfläche 25 gelegenen Meßelektrode 20 zugeordneten Ersatz-Widerstandschaltung 70 verbunden und aus einer oder mehreren - vorzugsweise, wie in Fig.13 gezeigt, zwei - nacheinander geschalteten Randzellen 71 gebildet, die nicht unmittelbar an Meßelektroden 20 angeschlossen sind. Die Messung der Temperatur der Abschluß-Zellen 71 erfolgt durch weitere Temperaturfühler, die in Fig.13 nicht gezeigt sind.

Die zwei Randzellen 71 weisen einen zentralen Knotenpunkt auf, der jeweils mit dem einen Anschluß von vier Horizontal-Randwiderständen 42', die jeweils gleich den Horizontal-Ersatzwiderständen 42 sind, und mit dem einen Anschluß von einem Vertikal-Randwiderstand 43 ', der gleich dem zweiten Vertikal-Ersatzwiderstand 43 ist, verbunden. Die anderen Enden der Horizontal-Randwiderstände 42' sind jeweils mit dem Horizontal-Ersatzwiderstand 42 bzw. dem Horizontal-Randwiderstand 42' der in Spalten- und Zeilenrichtung benachbarten Ersatz-Widerstandschaltungen 70 bzw. Randzellen 71 verbunden ist. Der andere Anschluß des Vertikal-Randwiderstandes 43' ist schließlich über den Blind-Widerstand 54 mit Masse verbunden.

Warum gerade zwei Randzellen 71 hintereinandergeschaltet sind, ergibt sich aus dem in der Praxis meßbaren Erwärmungseffekt, der bis zu einem Abstand in der Größenordnung von 2 cm festgestellt werden kann. Da jede Randzelle 71 sich in Normalrichtung zum Rand der Neutralelektrode über 1 cm erstreckt, ergibt sich daraus die Zellenzahl 2. Bei einer anderen Unterteilung der Meßfläche ändert sich die Zahl der zu kombinierenden Abschlußzellen 71 entsprechend.

Neben dem Randbereich um die Neutralelektrode ergibt sich in dem um diesen herum bestehenden Hautvolumen ebenso ein Stromfluß, der nicht zur Erwärmung beiträgt, dafür aber als Anteil am Gesamtstrom, den im Neutralelektrodenbereich fließenden Strom entsprechend mindert. Es hat sich herausgestellt, daß der durch die im Vergleich zur Neutralelektrodenfläche unendlich ausgedehnte Haut fließende Strom sich durch 15 bis 20 Randzellen geeignet simulieren läßt. Jede Randimpedanzschaltung 60 ist daher über eine Abschlußimpedanzschaltung 153, 154 mit Masse verbunden, wobei die Dimensionierung so erfolgt, daß durch diese ein den 15 bis 20 Randzellen entsprechender Strom fließt. Die Zahl der durch die Abschlußimpedanzschaltung, die in Fig.13 durch eine Serienschaltung aus einem Abschlußwiderstand 153 und einem Kondensator 154 gebildet ist, simulierten Randzellen kann im Rahmen der Erfindung je nach den Gegebenheiten variieren.

In den Ersatzwiderstandschaltungen gemäß Fig. 4 ist das Muskelgewebe durch eine Äquipotentialfläche nachgebildet, die durch die Verbindung der Vertikal-Ersatzwiderstände 44 mit Masse realisiert ist. Die Horizontal-Ersatzwiderstände Rh (Fig.3) werden im sehr gut leitenden Muskelgewebe mit 0 Ohm angenommen, wodurch die bereits weiter oben beschriebene Richtungsabhängigkeit im Modell nicht wiedergegeben wird, die sich durch stärkere Erwärmung des zur Operationsstelle näher gelegenen Randes der Neutralelektrode bemerkbar macht.

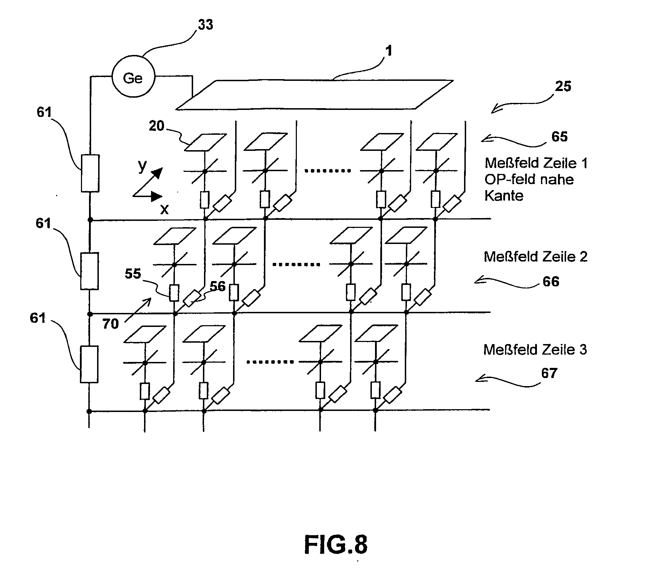

Für die vollständige elektronische Simulation der Richtungsabhängigkeit wären insgesamt vier weitere Ersatzwiderstände Rh für jede Meßelektrode 20 erforderlich. Aus Symmetriegründen fließen jedoch nur in einer horizontalen Richtung, z.B. in Richtung der Spalten (y-Richtung) Querströme zur Operationsstelle im Muskel, die Horizontal-Ersatzwiderstände in Richtung der Zeilen (x-Richtung) können daher entfallen und es können jeweils alle in einer Zeile der Meßelektroden-Matrix liegenden Horizontal-Ersatzwiderstände 56 zu einem Ersatzwiderstand 61 zusammengefaßt und dieser außerhalb des Meßbereiches der Temperaturfühler angeordnet werden, wie dies im Ausführungsbeispiel gemäß Fig.8 gezeigt ist, in der die Vertikalwiderstände 43, 54 als Impedanz 55 und die Horizontalwiderstände Rh des Muskelgewebes in Spaltenrichtung als Widerstände 56 dargestellt sind. Eine Spannungsquelle 33 zum Betrieb der erfindungsgemäßen Vorrichtung ist zwischen die Neutralelektrode 1 und die Horizontal-Ersatzwiderstände 61 geschaltet, welche die Horizontalwiderstände Rh des Muskelgewebes in Spaltenrichtung repräsentieren, außerhalb des räumlichen Meßbereiches der Temperaturfühler angeordnet sind und damit die Temperaturmessung nicht beeinflussen. Es kann im Rahmen der Erfindung x- und y-Richtung vertauscht sein, wobei dann die Auslagerung der Horizontalwiderstände Rh für die Zeilenrichtung der Meßelektroden-Matrix durchgeführt wird.

In Zusammenhang mit der Anwendung einer geteilten Neutralelektrode für elektrochirurgische Zwecke ist ein sogenannter CQM (Contact Quality Monitoring)-Test üblich, um die Qualität des Kontaktes zwischen der Haut des Patienten und der Neutralelektrode während der Operation zu bestimmen. Dabei wird beispielsweise eine Spannung an die beiden Hälften einer zweigeteilten Neutralelektrode angelegt und aus dem Stromfluß der Widerstand bestimmt. Es kann dieses Meßverfahren aber auch für mehrgeteilte Neutralelektroden Anwendung finden. Bisher mußte der Funktionstest des CQM aber immer an Versuchspersonen vorgenommen werden.

Eine Ausführungsform der Erfindung besteht in der Verwendung der erfindungsgemäßen Vorrichtung zur Überprüfung der . Neutralelektroden-Kontaktqualitätsüberwachungsschaltung (QCM) eines elektrochirurgischen Geräts. Die erfindungsgemäße Vorrichtung wird dabei passiv ohne Spannungsquelle betrieben und simuliert dabei die Impedanz eines Hautteilstückes. Zu diesem Zweck wird auf die erfindungsgemäße Vorrichtung eine zu überprüfende Neutralelektrode aufgebracht und dann der CQM-Test an dieser duchgeführt. Damit ist ein Test dieser Überwachungsschaltung unter kontrollierten Bedingungen möglich, wobei das aus den einzelnen Ersatz-Widerstandschaltungen und gegebenenfalls den Rand- und Abschlußimpedanzschaltungen zusammengesetzte Impedanz-Netzwerk als Testimpedanz verwendet wird.

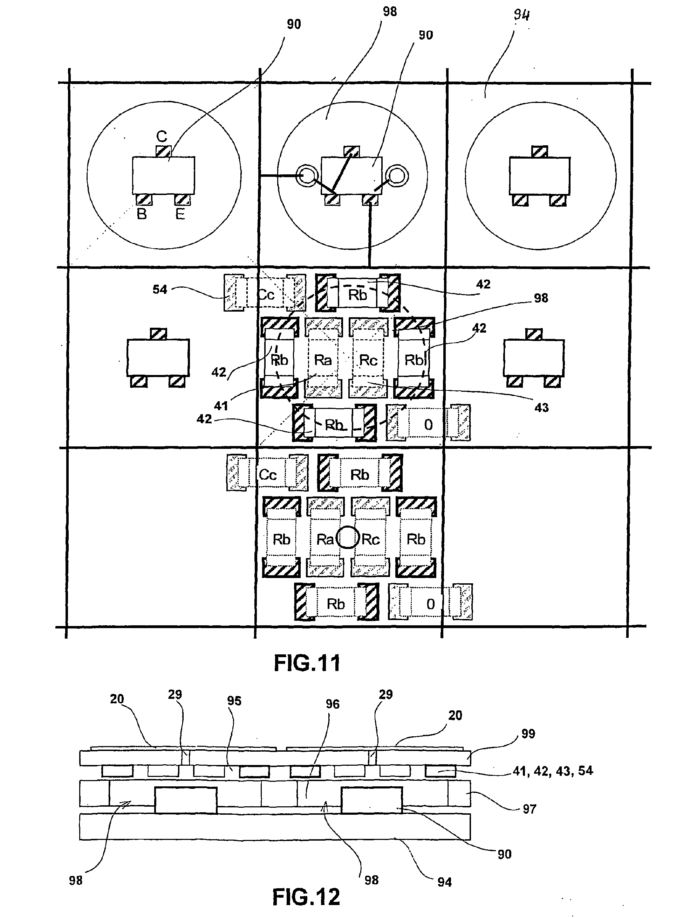

Für die Funktion der erfindungsgemäßen Vorrichtung ist die Anordnung der Widerstände der Ersatz-Widerstandschaltungen 70 von großer Bedeutung für die ordnungsgemäße Funktion. Eine bevorzugte Anordnung von Ersatzwiderständen und Temperaturfühlern ist in Fig.12 gezeigt. Die Meßelektroden 20 sind auf einer Seite einer die Meßfläche 25 bildenden Meßplatine 99 ausgebildet und die Widerstände 41, 42, 43, 54 der den Meßelektroden 20 zugeordneten Ersatz-Widerstandschaltungen 70 auf der gegenüberliegenden Seite der Meßplatine 99 angeordnet.

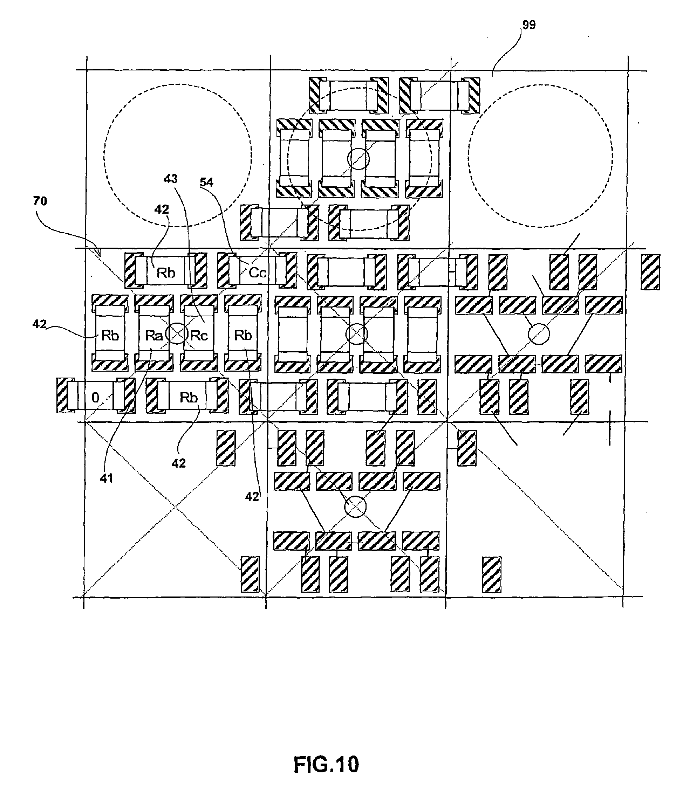

Erfindungsgemäß sind die Temperaturfühler 90 auf einer weiteren Platine 94 angeordnet, welche von der Meßplatine 99 durch eine mit Durchbrechungen 98 versehene Lochrasterplatte 97 beabstandet ist. Die den Widerständen 41, 42, 43, 54 der Ersatz-Widerstandschaltungen 70 zugekehrten Temperaturfühler 90 ragen dabei in die Durchbrechungen 98 der Lochrasterplatte 97. Aus den Layout-Darstellungen der Fig.10 und 11 ist erkennbar, daß die nicht dargestellten Meßelektroden 20 matrixartig und die Durchbrechungen 98 der Lochrasterplatte 97 konzentrisch zu den Meßelektroden-Mittelpunkten angeordnet sind. Der innerhalb der Durchbrechungen 98 bestehende Raum zwischen den Widerständen 41, 42, 43, 54 der Ersatz-Widerstandschaltungen 70 und den Temperaturfühlern 90 ist mit einem wärmeleitenden Material, vorzugsweise einer Wärmeleitpaste, gefüllt, die aufgrund ihrer Klebeeigenschaften die Meßplatine 99, die weitere Platine 94 und die Lochrasterplatte 97 zusammenhält und aufgrund ihrer guten Wärmeleitfähigkeit die von den Ersatzwiderständen 41, 42, 43 abgegebene Verlustleistung fast vollständig an die Temperaturfühler 90 weiterleitet und so eine Verfälschung des Meßergebnisses verhindern hilft.

Da die Ersatzwiderstände 41, 42, 43 auch seitlich Wärme an die unmittelbar benachbarten Ersatzwiderstände abgeben, ist vorzugsweise der Zwischenraum zwischen den Ersatzwiderständen 41, 42, 43, 54 der Ersatz-Widerstandschaltungen 70 mit einem wärmeisolierenden Material gefüllt.

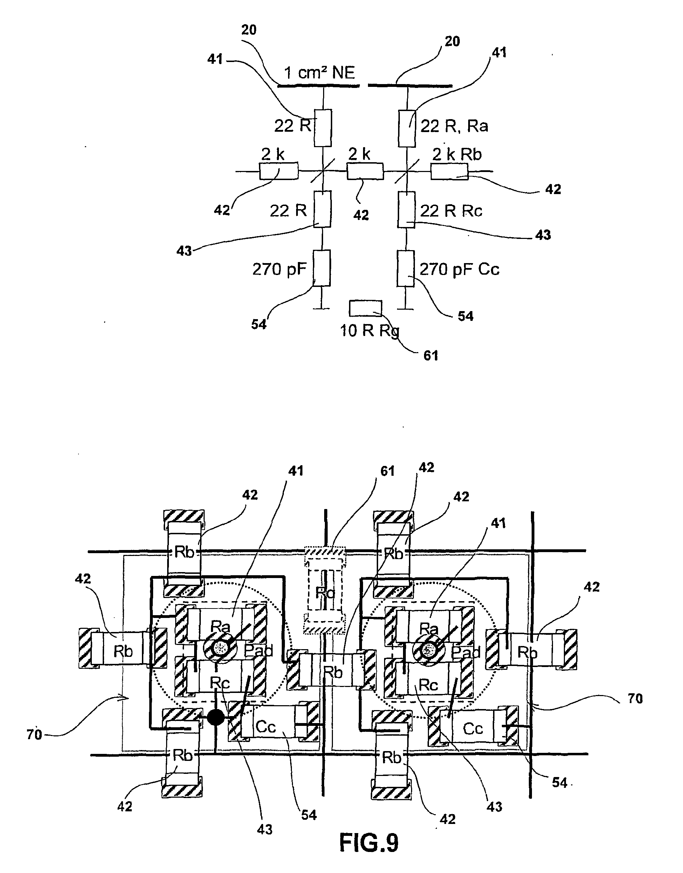

Bevorzugt werden die Ersatzwiderstände 41, 42, 43, 54 der Ersatz-Widerstandschaltungen 70 und die Temperaturfühler 90 durch SMD-Bauelemente gebildet, wie aus den Fig.9 bis 11 erkennbar ist. Die in Fig.9 gezeigte Ausführungsform hat den Nachteil, daß die Ersatzwiderstände 42 relativ weit von den Temperaturfühlerpositionen entfernt angeordnet sind und bewirken damit für jeweils benachbarte Meßelektroden 20 ebenfalls eine Erwärmung. Eine Verbesserung dieser Situation ergibt sich durch die in Fig.10 und 11 getroffene Anordnung der Ersatzwiderstände der Ersatz-Widerstandschaltungen 70 im unmittelbaren Bereich der zugehörigen Meßelektrode 20 auf der gegenüberliegenden Seite der Meßplatine 99. Über jeweils zumindest eine Durchkontaktierung 29 ist jeweils die zugehörige Meßelektrode 20 mit der Ersatz-Widerstandschaltung 70 verbunden. Durch die dichte Packung der Ersatzwiderstände jeweils im Bereich der Meßelektrode 20 ist eine nur geringe Beeinflussung von benachbarten Ersatz-Widerstandschaltungen 70 gegeben.

## Patentansprüche

1. Vorrichtung zur Prüfung einer Neutralelektrode (1) für elektrochirurgische Anwendungen mit einer aus einer Vielzahl von Meßelektroden (20) gebildeten Meßfläche (25), auf welche die Neutralelektrode (1) aufbringbar ist, wobei jede Meßelektrode (20) der Meßfläche (25) mit einer zumindest eine Schicht der menschlichen Haut, z.B. die Oberhaut, die Lederhaut und die Unterhaut mit Fettgewebe, repräsentierenden Ersatz-Widerstandschaltung (70) aus zumindest zwei Widerständen (41, 42, 43, 54) verbunden ist, welche Ersatz-Widerstandschaltung (70) mit zumindest jeweils einem Temperaturfühler (90) in thermischem Kontakt steht, **dadurch gekennzeichnet, daß** zumindest eine Hautschicht innerhalb der Ersatz-Widerstandschaltung (70) von einem Blindwiderstand (54) repräsentiert wird.

2. Vorrichtung nach Anspruch 1, **dadurch gekennzeichnet, daß** der Blindwiderstand durch eine Kapazität (54) gebildet ist.

3. Vorrichtung nach Anspruch 1 oder 2, **dadurch gekennzeichnet, daß** der die Unterhaut repräsentierende Widerstand der Ersatz-Widerstandschaltung (70) durch den Blindwiderstand (54) gebildet ist.

4. Vorrichtung nach Anspruch 1, 2 oder 3, welche bei aufgebrachter Neutralelektrode (1) mit einer Wechselspannungsquelle (3) verbindbar ist, **dadurch gekennzeichnet, daß** die Wechselspannungsquelle (3) variable Frequenz aufweist.

5. Vorrichtung nach einem der Ansprüche 1 bis 4, **dadurch gekennzeichnet, daß** die Meßelektroden (20) mit den ihr zugeordneten Ersatz-Widerstandschaltungen (70) in Form einer Matrix mit Spalten und Zeilen (65, 66, 67) angeordnet sind

6. Vorrichtung nach Anspruch 5, **dadurch gekennzeichnet, daß** jede Ersatz-Widerstandschaltung (70) einen zentralen Knotenpunkt aufweist, der jeweils mit dem einen Anschluß von vier Horizontal-Ersatzwiderständen (42) und mit dem einen Anschluß von einem ersten und einem zweiten Vertikal-Ersatzwiderstand (41, 43) verbunden ist, wobei das andere Ende der Horizontal-Ersatzwiderstände (42) jeweils mit dem Horizontal-Ersatzwiderstand (42) der in Spalten- und Zeilenrichtung benachbarten Ersatz-Widerstandschaltungen (70) verbunden ist, und wobei der andere Anschluß des ersten Vertikal-Ersatzwiderstandes (41) mit der Meßelektrode (20) und der andere Anschluß des zweiten Vertikal-Ersatzwiderstandes (43) über den Blind-Widerstand (54) mit Masse verbunden ist.

7. Vorrichtung nach Anspruch 5 oder 6, **dadurch gekennzeichnet, daß** die den entlang des Randes der Meßfläche (25) angeordneten Meßelektroden (20) zugeordneten Ersatz-Widerstandschaltungen (70) jeweils mit einer Randimpedanzschaltung (60) mit zumindest einem zugeordneten Temperaturfühler verbunden sind.

8. Vorrichtung nach Anspruch 7, **dadurch gekennzeichnet, daß** die Randimpedanzschaltung (60) jeweils mit dem Anschluß des randseitig offenen Horizontal-Ersatzwiderstandes (42) der der am Rand der Meßfläche (25) gelegenen Meßelektrode (20) zugeordneten Ersatz-Widerstandschaltung (70) verbunden ist.

9. Vorrichtung nach Anspruch 7 oder 8, **dadurch gekennzeichnet, daß** jede Randimpedanzschaltung (60) aus einer oder mehreren, vorzugsweise zwei, nacheinander geschalteten Randzellen (71) gebildet ist, wobei die Randzelle(n) (71) jeweils einen zentralen Knotenpunkt aufweist bzw. aufweisen, der jeweils mit dem einen Anschluß von vier Horizontal-Randwiderständen (42'), die jeweils gleich den Horizontal-Ersatzwiderständen (42) sind, und mit dem einen Anschluß von einem Vertikal-Randwiderstand (43'), der gleich dem zweiten Vertikal-Ersatzwiderstand (43) ist, verbunden ist, wobei das andere Ende der Horizontal-Randwiderstände (42') jeweils mit dem Horizontal-Ersatzwiderstand (42) bzw. dem Horizontal-Randwiderstand (42') der in Spalten- und Zeilenrichtung benachbarten Ersatz-Widerstandschaltungen (70) bzw. Randzellen (71) verbunden ist, und wobei der andere Anschluß des Vertikal-Randwiderstandes (43') über den Blind-Widerstand (54) mit Masse verbunden ist.

10. Vorrichtung nach einem der Ansprüche 5 bis 9, **dadurch gekennzeichnet, daß** jede Randimpedanzschaltung (60) über eine Abschlußimpedanzschaltung (153, 154) mit Masse verbunden ist.

11. Vorrichtung nach einem der Ansprüche 5 bis 10, **dadurch gekennzeichnet, daß** für jede Zeile (65, 66, 67) oder für jede Spalte der Matrix aus Meßelektroden (20) und zugeordneten Ersatz-Widerstandschaltungen jeweils ein gemeinsamer, die Muskelschicht repräsentierender Horizontal-Ersatzwiderstand (61) vorgesehen ist, der außerhalb des räumlichen Meßbereiches der Temperaturfühler (90) angeordnet ist.

12. Vorrichtung nach Anspruch 11, **dadurch gekennzeichnet, daß** ein Anschluß der gemeinsamen Horizontal-Ersatzwiderstände (61) jeweils mit den den Meßelektroden (20) der Zeile (65, 66, 67) oder der Spalte zugeordneten Ersatz-Widerstandschaltungen (70) verbunden ist.

13. Vorrichtung nach einem der vorhergehenden Ansprüche, **dadurch gekennzeichnet, daß** die mit den Ersatz-Widerstandschaltungen (70) in thermischem Kontakt stehenden Temperaturfühler in an sich bekannter Weise durch Bipolartransistoren (90) gebildet sind.

14. Verwendung einer Vorrichtung nach einem der Ansprüche 1 bis 13 zur Überprüfung der Neutralelektroden-Kontaktqualitätsüberwachungsschaltung (CQM) eines elektrochirurgischen Geräts.

15. Vorrichtung nach einem der vorhergehenden Ansprüche, **dadurch gekennzeichnet, daß** die Meßelektroden (20) auf einer Seite einer die Meßfläche (25) bildenden Meßplatine (99) ausgebildet und die Widerstände (41, 42, 43, 54) der zugeordneten Ersatz-Widerstandschaltungen (70) auf der gegenüberliegenden Seite der Meßplatine (99) angeordnet sind, und daß die Temperaturfühler (90) auf einer weiteren Platine (94) angeordnet sind, welche von der Meßplatine (99) durch eine mit Durchbrechungen (98) versehene Lochrasterplatte (97) beabstandet ist, wobei die den Widerständen (41; 42, 43; 54) der Ersatz-Widerstandschaltungen (70) zugekehrten Temperaturfühler (90) in die Durchbrechungen (98) der Lochrasterplatte (97) ragen.

16. Vorrichtung nach Anspruch 15, **dadurch gekennzeichnet, daß** die Meßelektroden (20) matrixartig angeordnet sind, und daß die Durchbrechungen (98) der Lochrasterplatte (97) konzentrisch zu den Meßelektroden-Mittelpunkten angeordnet sind.

17. Vorrichtung nach Anspruch 15 oder 16, **dadurch gekennzeichnet, daß** der innerhalb der Durchbrechungen (98) bestehende Raum zwischen den Widerständen (41, 42, 43, 54) der Ersatz-Widerstandschaltungen (70) und den Temperaturfühlern (90) mit einem wärmeleitenden Material gefüllt ist.

18. Vorrichtung nach Anspruch 15, 16 oder 17, **dadurch gekennzeichnet, daß** der Zwischenraum zwischen den Widerständen (41, 42, 43, 54) der Ersatz-Widerstandschaltungen (70) mit einem wärmeisolierenden Material gefüllt ist.

19. Vorrichtung nach einem der Ansprüche 15 bis 18, **dadurch gekennzeichnet, daß** die Widerstände (41, 42, 43, 54) der Ersatz-Widerstandschaltungen (70) und die Temperaturfühler (90) durch SMD-Bauelemente gebildet sind.

20. Vorrichtung nach einem der Ansprüche 15 bis 19, **dadurch gekennzeichnet, daß** die Widerstände (41, 42, 43, 54) der Ersatz-Widerstandschaltungen (70) im unmittelbaren Bereich der zugehörigen Meßelektrode (20) auf der gegenüberliegenden Seite der Meßplatine (99) angeordnet und über jeweils zumindest eine Durchkontaktierung (29) mit dieser verbunden sind.

## Claims

1. A device for testing a neutral electrode (1) for use in electrosurgery, comprising a measuring surface (25) which is formed by a plurality of measuring electrodes (20), whereon said neutral electrode (1) may be applied, wherein each measuring electrode (20) of the measuring surface (25) is connected to an equivalent resistance circuit (70) representing at least one layer of the human skin, e.g. the epidermis, the corium and the hypodermis including fatty tissue, and consisting of at least two resistances (41, 42, 43, 54), which equivalent resistance circuit (70) is in thermal contact with at least one temperature sensor (90), **characterised in that** at least one skin layer within the equivalent resistance circuit (70) is represented by a reactance (54).

2. The device according to claim 1, **characterised in that** said reactance is formed by a capacitance (54).

3. The device according to claim 1 or 2, **characterised in that** the resistance of the equivalent resistance circuit (70) representing the hypodermis is formed by said reactance (54).

4. The device according to claim 1, 2, or 3, which is connectable to an alternating voltage source (3) when the neutral electrode (1) is applied, **characterised in that** said alternating voltage source (3) has a variable frequency.

5. The device according to any one of claims 1 to 4, **characterised in that** the measuring electrodes (20) and the respective associated equivalent resistance circuits (70) are arranged in the form of a matrix of columns and rows (65, 66, 67).

6. The device according to claim 5, **characterised in that** each equivalent resistance circuit (70) has a central node which is connected to one terminal each of four horizontal equivalent resistances (42) and to one terminal each of a first and a second vertical equivalent resistance (41, 43), wherein the other ends of the horizontal equivalent resistances (42) are connected to the horizontal equivalent resistances (42) of the equivalent resistance circuits (70) adjacent in the column and row directions, and wherein the other terminal of the first vertical equivalent resistance (41) is connected to the measuring electrode (20) and the other terminal of the second vertical equivalent resistance (43) is connected to ground via the reactance (54).

7. The device according to claim 5 or 6, **characterised in that** the equivalent resistance circuits (70) associated with the measuring electrodes (20) arranged along the periphery of the measuring surface (25) are each connected to a peripheral impedance circuit (60) with at least one associated temperature sensor.

8. The device according to claim 7, **characterised in that** each peripheral impedance circuit (60) is connected to the terminal of the horizontal equivalent resistance (42), which is unconnected at the periphery, of the equivalent resistance circuit (70) associated with the measuring electrode (20) situated at the periphery of the measuring surface (25).

9. The device according to claim 7 or 8, **characterised in that** each peripheral impedance circuit (60) consists of one or more, preferably two, serially connected peripheral cells (71), wherein each peripheral cell (71) has a central node connected to one terminal each of four horizontal peripheral resistances (42'), which correspond to the horizontal equivalent resistances (42), and to the one terminal of a vertical peripheral resistance (43'), which corresponds to the second vertical equivalent resistance (43), wherein the other ends of the horizontal peripheral resistances (42') are each connected to the horizontal equivalent resistance (42) or the horizontal peripheral resistance (42') of the equivalent resistance circuits (70) or peripheral cells (71) adjacent in the column and row directions, and wherein the other terminal of the vertical peripheral resistance (43') is connected to ground via the reactance (54).

10. The device according to any one of the claims 5 to 9, **characterised in that** each peripheral impedance circuit (60) is connected to ground via a terminal impedance circuit (153, 154).

11. The device according to any one of the claims 5 to 10, **characterised in that** for each row (65, 66, 67) or each column of the matrix consisting of measuring electrodes (20) and associated equivalent resistance circuits a common horizontal equivalent resistance (61) representing the muscle layer is provided, which is arranged outside the spatial measuring range of the temperature sensor (90).

12. The device according to claim 11, **characterised in that** one terminal of each of the common horizontal equivalent resistances (61) is connected to equivalent resistance circuits (70) associated with the measuring electrodes (20) of the row (65, 66, 67) or of the column.

13. The device according to any one of the preceding claims, **characterised in that** the temperature sensors in thermal contact with the equivalent resistance circuits (70) are formed in a generally known manner by bipolar transistors (90).

14. Use of a device according to any one of the claims 1 to 13 for monitoring the neutral electrode contact quality monitoring circuit (CQM) of an electrosurgical apparatus.

15. The device according to any one of the preceding claims, **characterised in that** the measuring electrodes (20) are provided on one side of a measuring circuit board (99) forming the measuring surface (25) and the resistances (41, 42, 43, 54) of the associated equivalent resistance circuits (70) are arranged on the opposite side of the measuring circuit board (99) and that the temperature sensors (90) are arranged on a further circuit board (94) which is spaced apart from said measuring circuit board (99) by a drilled board (97) provided with through holes (98), wherein the temperature sensors (90) facing the resistances (41, 42, 43, 54) of the equivalent resistance circuits (70) project into said through holes (98) of said drilled board (97).

16. The device according to claim 15, **characterised in that** the measuring electrodes (20) are arranged in the form of a matrix and that said through holes (98) of said drilled board (97) are arranged concentrically with the centres of the measuring electrodes.

17. The device according to claim 15 or 16, **characterised in that** the space between the resistances (41, 42, 43, 54) of the equivalent resistance circuits (70) and the temperature sensors (90) is filled with a heat-conducting material.

18. The device according to claim 15, 16, or 17, **characterised in that** the space between the resistances (41, 42, 43, 54) of the equivalent resistance circuits (70) is filled with a heat-insulating material.

19. The device according to any one of the claims 15 to 18, **characterised in that** the resistances (41, 42, 43, 54) of the equivalent resistance circuits (70) and the temperature sensors (90) are formed by SMD components.

20. The device according to any one of the claims 15 to 19, **characterised in that** the resistances (41, 42, 43, 54) of the equivalent resistance circuits (70) are arranged in the immediate vicinity of the associated measuring electrode (20) on the opposite side of the measuring circuit board (99) and are connected to it via at least one feedthrough (29).

## Revendications

1. Dispositif de test d'une électrode neutre (1) pour des applications électrochirurgicales comprenant une surface de mesure (25) constituée par une pluralité d'électrodes de mesure (20), sur laquelle ladite électrode neutre (1) peut être appliquée, chaque électrode de mesure (20) de la surface de mesure (25) étant connectée avec un circuit équivalent de résistance (70) représentant au moins une couche de la peau humaine, p.ex. l'épiderme, la sclérotique et l'hypoderme avec le tissu adipeux, et comprenant au moins deux résistances (41, 42, 43, 54), ce circuit équivalent de résistance (70) étant en contact thermique avec au moins une sonde de température (90), **caractérisé en ce que** au moins une couche de peau dans ledit circuit équivalent de résistance (70) est constituée par une réactance (54).

2. Dispositif selon la revendication 1, **caractérisé en ce que** ladite réactance (54) est constituée par une capacitance (54).

3. Dispositif selon la revendication 1 ou 2, **caractérisé en ce que** la résistance du circuit équivalent de résistance (70) représentant l'hypoderme est constituée par ladite réactance (54).

4. Dispositif selon la revendication 1, 2 ou 3, qui peut être connecté avec une source de tension alternative (3) quand l'électrode neutre (1) est appliquée, **caractérisé en ce que** ladite source de tension alternative (3) a une fréquence variable.

5. Dispositif selon l'une des revendications 1 à 4, **caractérisé en ce que** lesdites électrodes de mesure (20) et les circuits équivalents de résistance (70) associés respectifs sont arrangées en forme d'une matrice de colonnes et de lignes (65, 66, 67).

6. Dispositif selon la revendication 5, **caractérisé en ce que** chaque circuit équivalent de résistance (70) dispose d'un noeud central qui est connecté avec un raccordement de chacune de quatre résistances équivalentes horizontales (42) et avec un raccordement de chacune d'une première et d'une deuxième résistance équivalente verticale (41, 43), l'autre bout de chaque résistance équivalente horizontale (42) étant connecté respectivement avec les circuits équivalents de résistance (70) contigus dans la direction des colonnes et des lignes et l'autre raccordement de la première résistance équivalente verticale (41) étant connecté avec l'électrode de mesure (20) et l'autre raccordement de la deuxième résistance équivalente verticale (43) étant relié à terre via la réactance (54).

7. Dispositif selon la revendication 5 ou 6, **caractérisé en ce que** les circuits équivalents de résistance (70) associés aux électrodes de mesure (20) arrangées le long du bord de la surface de mesure (25) sont respectivement connectés avec un circuit d'impédance marginal (60) avec au moins une sonde de température associée.

8. Dispositif selon la revendication 7, **caractérisé en ce que** chaque circuit d'impédance marginal (60) est connecté avec le raccordement de la résistance équivalente horizontale (42), qui n'est pas connectée au bord, du circuit équivalent de résistance (70) associé à l'électrode de mesure (20) située au bord de la surface de mesure (25).

9. Dispositif selon la revendication 7 ou 8, **caractérisé en ce que** chaque circuit d'impédance marginal (60) est constitué par une ou plusieurs, de préférence deux, cellules marginales (71) connectées en série, chaque cellule marginale (71) disposant d'un noeud central qui est connecté avec un raccordement de chacune de quatre résistances marginales horizontales (42'), correspondant aux résistances équivalentes horizontales (42), et avec un raccordement d'une résistance marginale verticale (43'), correspondant à la deuxième résistance équivalente verticale (43), l'autre bout de chaque résistance marginale horizontale (42') étant connecté respectivement avec la résistance équivalente horizontale (42) ou la résistance marginale horizontale (42') des circuits équivalents de résistance (70) ou des cellules marginales (71) contigus dans la direction des colonnes et des lignes et l'autre raccordement de la résistance marginale verticale (43') étant relié à terre via la réactance (54).

10. Dispositif selon l'une des revendications 5 à 9, **caractérisé en ce que** chaque circuit d'impédance marginal (60) est relié à terre via un circuit d'impédance terminal (153, 154).

11. Dispositif selon l'une des revendications 5 à 10, **caractérisé en ce que** pour chaque ligne (65, 66, 67) ou pour chaque colonne de la matrice constituée par les électrodes de mesure (20) et les circuits équivalents de résistance associés une résistance équivalente horizontale (61) commune représentant la couche musculaire est prévue, cette résistance équivalente horizontale (61) étant arrangée hors de la zone spatiale de mesure de la sonde de température (90).

12. Dispositif selon la revendication 11, **caractérisé en ce qu'**un raccordement de chaque résistance équivalente horizontale (61) commune est connecté avec les circuits équivalents de résistance (70) associés aux électrodes de mesure (20) de la ligne (65, 66, 67) ou de la colonne.

13. Dispositif selon l'une des revendications précédentes, **caractérisé en ce que** les sondes de température, qui sont en contact thermique avec les circuits équivalents de résistance (70), sont constituées, d'une manière connue, par des transistors bipolaires (90).

14. Utilisation d'un dispositif selon l'une des revendications 1 à 13 pour le contrôle du circuit de contrôle de la qualité du contact (CQM) de la électrode neutre d'un instrument électrochirurgical.

15. Dispositif selon l'une des revendications précédentes, **caractérisé en ce que** les électrodes de mesure (20) sont formées sur une côté d'une platine de mesure (99) constituant la surface de mesure (25) et que les résistances (41, 42, 43, 54) des circuits équivalents de résistance (70) associés sont arrangées sur la côté opposée de la platine de mesure (99) et que la sonde de température (90) est arrangé sur une autre platine (94) qui est écartée de ladite platine de mesure (99) par un panneau perforé (97) fournis des orifices (98), les sondes de température (90) tournées vers les résistances (41, 42, 43, 54) des circuits équivalents de résistance (70) s'élevant dans lesdites orifices (98) dudit panneau perforé (97).

16. Dispositif selon la revendication 15, **caractérisé en ce que** les électrodes de mesure (20) sont arrangées en forme d'une matrice et que les orifices (98) du panneau perforé (97) sont arrangés d'une manière concentrique avec les centres des électrodes de mesure.

17. Dispositif selon la revendication 15 ou 16, **caractérisé en ce que** l'espace dans les orifices (98) entre les résistances (41, 42, 43, 54) des circuits équivalents de résistance (70) et les sondes de température (70) est rempli d'un matériau thermoconducteur.

18. Dispositif selon la revendication 15, 16 ou 17, **caractérisé en ce que** l'espace entre les résistances (41, 42, 43, 54) des circuits équivalents de résistance (70) est rempli d'un matériau thermo-isolant.

19. Dispositif selon l'une des revendications 15 à 18, **caractérisé en ce que** les résistances (41, 42, 43, 54) des circuits équivalents de résistance (70) et les sondes de température (90) sont constituées par des composants SMD.

20. Dispositif selon l'une des revendications 15 à 19, **caractérisé en ce que** les résistances (41, 42, 43, 54) des circuits équivalents de résistance (70) sont arrangées dans les environs immédiats de la électrode de mesure (20) associée respective sur la côté opposée de la platine de mesure (99) et sont connectées avec celle-ci via au moins une connexion transversale (29).
